(19) Europäisches Patentamt
European Patent Office
Office européen des brevets

(11) EP 0 925 774 A2

(12) EUROPÄISCHE PATENTANMELDUNG

(43) Veröffentlichungstag:
30.06.1999 Patentblatt 1999/26

(51) Int. Cl.$^6$: **A61K 7/00**, A61K 7/11,
A61K 7/06

(21) Anmeldenummer: 98121674.0

(22) Anmeldetag: 13.11.1998

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 06.12.1997 DE 19754270

(71) Anmelder:
Wella Aktiengesellschaft
64274 Darmstadt (DE)

(72) Erfinder:
• Schmenger, Jürgen
  Woodland Hills, CA 91367 (US)
• Wendel, Harald
  64372 Ober-Ramstadt (DE)
• Steinbrecht, Karin Dr.
  64372 Ober-Ramstadt (DE)

(54) **Verwendung von Polymeren natürlichen Ursprungs zur Verbesserung von Polyurethanfilmen**

(57)     Die Erfindung betrifft die Verwendung von Polymeren natürlichen Ursprungs zur Verbesserung von Filmbildungseigenschaften von Polyurethanen sowie Haarbehandlungsmittel mit einem Gehalt an

(A) mindestens einem Polyurethan und
(B) mindestens einem Polymer natürlichen Ursprungs.

Durch die erfindungsgemäße Kombination der Komponenten (A) und (B) werden die Nachteile von Polyurethanen ausgeglichen und bei einem Einsatz in wässrigen oder wässrig-alkoholischen Haarsprays die Eigenschaften von rein alkoholischen Haarsprays erreicht und zum Teil übertroffen, was durch keines der Polymere allein möglich ist.

EP 0 925 774 A2

**Beschreibung**

Gegenstand der Erfindung ist eine neue Verwendung von Polymeren natürlichen Ursprungs sowie ein Haarbehandlungsmittel mit einem Gehalt an mindestens einem Polyurethan sowie einem Gehalt an mindestens einem Polymer natürlichen Ursprungs.

Haarbehandlungsmittel mit haarfestigender Wirkung wie beispielsweise Haarsprays wurden und werden üblicherweise in Form von Polymerlösungen in alkoholischem Milieu verwendet. Aufgrund von Umweltschutzgesichtspunkten sowie zu erwartender gesetzlicher Reglementierungen besteht ein Bedarf, den Gehalt an leicht flüchtigen organischen Bestandteilen (VOC, volatile organic compounds), zu denen u.a. auch die bisher in Haarsprays üblichen alkoholischen Lösungsmittel gehören, möglichst weit zu reduzieren. Haarbehandlungsmittel mit festigender Wirkung in Form von Polymerlösungen in wässrigem oder wässrig-alkoholischem Milieu mit reduziertem Alkoholgehalt sind bereits bekannt. Hierfür werden in der Regel neu entwickelte Polymere eingesetzt, die speziell für wässrig-alkoholische Lösungsmittelsysteme entwickelt wurden.

So sind beispielsweise wässrige oder alkoholische Dispersionen oder Lösungen von Polyurethanen verschiedenster Art geeignet, in sogenannten VOC-Haarsprays (Haarsprays mit einem reduziertem Anteil an leicht flüchtigen organischen Bestandteilen) eingesetzt zu werden. Aufgrund ihrer Festigungseigenschaften im vollständig getrockneten Zustand, ihrer geringen Eigenviskosität in Dispersionen, der guten Treibgas- und Kältestabilität besitzen Polyurethane eine Reihe von wichtigen Eigenschaften für eine Verwendung in VOC-Haarsprays. Geeignete Polyurethane werden beispielsweise in der WO 97/17386, der US 5,626,840 und in der WO 96/14049 beschrieben. Die Nachteile der Polyurethane sind eine lange Trocknungsdauer, ein zu weicher, glatter Griff des Polymerfilms auf dem Haar und ungenügende Vernetzungs- und Festigungseigenschaften des Polymerfilmes auf Haaren.

Es war aber bisher nicht möglich, wässrige oder wässrig-alkoholische Haarsprays herzustellen, die hinsichtlich der üblicherweise an ein Haarspray zu stellenden Qualitätsanforderungen gleichwertig sind mit rein alkoholischen Haarsprays. Die Hauptkriterien Haarfestigung, Haarvernetzung, Griff (Rauhigkeit, Klebrigkeit, Glattheit) und Trockenzeit der wässrigen oder wässrig-alkoholischen Haarsprays wurden sowohl von Endverbrauchern als auch von Friseuren bisher immer als unakzeptabel schlechter beurteilt.

Es ist aus der WO 97/17386 zwar bekannt, daß die dort beschriebenen Polyurethane mit weiteren, bekannten synthetischen Polymeren kombiniert werden können. Die typischen, negativen Eigenschaften der Polyurethane wie beispielsweise ungenügende Festigungsleistung, schlechte Vernetzungseigenschaften und zu weiches Anfühlen des Polymerfilms (Griff auf dem Haar) können durch die Kombination mit den in der WO 97/17386 genannten zusätzlichen synthetischen Polymeren jedoch nicht behoben werden. Es kommt hierbei teilweise zu Schaumbildungen beim Versprühen, Trübungen und Ausfällungen der Lösungen oder zu allgemeinen Unverträglichkeiten, die es nicht erlauben, ein den handelsüblichen, alkoholischen Haarsprays vergleichbares, den Verbrauchergewohnheiten entsprechendes wäßrig-alkoholisches Haarspray herzustellen.

Es bestand daher die Aufgabe, die Eigenschaften von aus Polyurethanen gebildeten Filmen zu verbessern, insbesondere im Hinblick auf die Herstellung eines Haarbehandlungsmittels mit festigender Wirkung, einem reduziertem Anteil an VOC-Bestandteilen und einer Qualität, die an diejenige von rein alkoholischen Haarsprays heranreicht.

Es wurde nun gefunden, daß die Aufgabe durch einen Zusatz an Polymeren natürlichen Ursprungs gelöst wird.

Gegenstand der Erfindung ist daher die Verwendung von Polymeren natürlichen Ursprungs zur Verbesserung der Filmbildungseigenschaften von Polyurethanen sowie zur Herstellung von Polyurethan enthaltenden Faserbehandlungsmitteln, insbesondere Keratin- und Haarbehandlungsmitteln.

Ein weiterer Gegenstand der vorliegenden Erfindung ist ein Haarbehandlungsmittel mit einem Gehalt an

(A) mindestens einem Polyurethan und
(B) mindestens einem Polymer natürlichen Ursprungs.

In dem erfindungsgemäßen Mittel werden die Polyurethane vorzugsweise in Form ihrer wäßrigen Lösungen oder Dispersionen eingesetzt.

Das Mittel enthält vorzugsweise 0,1 bis 20 Gewichtsprozent, besonders bevorzugt 0,5 bis 10 Gewichtsprozent der Komponente (A) und vorzugsweise 0,1 bis 20 Gewichtsprozent, besonders bevorzugt 0,5 bis 10 Gewichtsprozent der Komponente (B).

Unter Polymeren natürlichen Ursprungs im Sinne der vorliegenden Erfindung sind solche Polymere, Oligomere oder Polymer- oder Oligomergemische zu verstehen, die ohne weitere chemische Modifikation aus natürlichem Material isoliert werden wie beispielsweise Polysaccharide, Oligosaccharide, Schellack, Chitine oder chinesisches Balsamharz (Kolophonium). Zu den Polymeren natürlichen Ursprungs im Sinne der vorliegenden Erfindung zählen außerdem natürliche Polymere die chemisch behandelt wurden um beispielsweise ihre Wasserlöslichkeit oder Wasserdispergierbarkeit zu erhöhen. Hierzu gehören beispielsweise hydrolysierte, hydroxylierte oder alkoxylierte Derivate wie beispielsweise Chitosan oder alkoxylierte Chitosane und alkoxylierte Chitine.

Bevorzugte Polymere natürlichen Ursprungs sind solche, die selber filmbildende und haarfestigende Eigenschaften aufweisen. Unter filmbildenden, haarfestigenden Polymeren werden erfindungsgemäß solche Polymere verstanden, die bei Anwendung in 0,01 bis 5%-iger wäßriger, alkoholischer oder wäßrig-alkoholischer Lösung in der Lage sind, auf dem Haar einen Polymerfilm abzuscheiden und auf diese Weise das Haar zu festigen.

Ein besonders bevorzugtes Polymer natürlichen Ursprungs ist handelsüblicher Schellack. Schellack wird vorzugsweise in zu 50 bis 100 Prozent neutralisierter Form und in einer Konzentration von 0,5 bis 5 Gewichtsprozent eingesetzt. Als Neutralisationsmittel für Schellack können beispielsweise primäre, sekundäre oder tertiäre Amine, Aminoalkohole, Alkalihydroxide, Ammoniak in Form von wässrigen Ammoniaklösungen oder Natriumtetraborat verwendet werden. Bevorzugte Neutralisationsmittel sind 2-Amino-2-methylpropanol, Triisopropylamin, Natriumhydroxid oder wässrige Ammoniaklösung.

Ein weiteres geeignetes Polymer ist niedermolekulares Chitosan mit einem Molekulargewicht von 30.000 bis 70.000 g/mol. Als Neutralisationsmittel für Chitosan können organische oder anorganische Säuren, vorzugsweise Ameisensäure, Pyrrolidoncarbonsäure oder Milchsäure verwendet werden.

Weiterhin können verschiedene Saccharidtypen verwendet werden wie zum Beispiel Polysaccharide oder Gemische aus Oligo-, Mono- und Disacchariden, welche beispielsweise unter dem Handelsnamen C-Pur$^{®}$ von der Firma Cerestar/Belgien vertrieben werden.

Bei den Polyurethanen kann es sich um wasserlösliche oder um wasserunlösliche, wasserdispergierbare Polyurethane handeln. Die Polyurethane bestehen aus mindestens zwei unterschiedlichen Monomertypen 1 und 2, wobei Monomertyp 1 mindestens zwei aktive Wasserstoffatome aufweist und Monomertyp 2 ein Di- oder ein Polyisocyanat ist. Darüberhinaus können die Polyurethane weitere kettenverlängernde Bausteine enthalten. Monomertyp 1 kann beispielsweise ein Diol, Triol, Polyetherol, Polyesterol, Diamin oder Triamin sein. Hierzu zählen u.a. Ethylenglykol, die Propylen- und Butylenglykole, Di-, Tri-, Tetra- und Polyethylen- und -propylenglykole, Copolymere von Alkylenoxiden wie Ethylenoxid, Propylenoxid oder Butylenoxid, Ethylendiamin, Propylendiamin, 1,4-Diaminobutan, Hexamethylendiamin und $\alpha,\omega$-Diamine von langkettigen Alkanen oder Polyalkylenoxiden. Polyesterole werden üblicherweise aus Di- oder Polyolen und Dicarbonsäuren wie Phtalsäure, Isophtalsäure oder Adipinsäure erhalten. Monomere mit mehr als zwei aktiven Wasserstoffatomen werden üblicherweise nur in geringen Mengen in Kombination mit einem großen Überschuß an Monomeren mit nur zwei aktiven Wasserstoffatomen eingesetzt.

Monomertyp 2 kann beispielsweise Hexamethylendiisocyanat, 2,4- und 2,6-Toluoldiisocyanat, 4,4'-Methylendi(phenylisocyanat) oder Isophorondiisocyanat sein.

Weitere Informationen zum Aufbau und zur Herstellung von Polyurethanen können den einschlägigen Nachschlagewerken wie Römpps Chemie-Lexikon oder Ullmanns Enzyklopädie der technischen Chemie entnommen werden.

Erfindungsgemäß bevorzugt einzusetzende Polyurethane und deren Herstellung sind insbesondere in der WO 97/17386 und in der US 5,626,840 beschrieben.

Bevorzugte Polyurethane sind dadurch gekennzeichnet, daß Sie (a) endständige Säuregruppen besitzen, die beispielweise über Aminosulfonsäuren oder Aminocarbonsäuren eingeführt wurden, (b) weitere freie Carbonsäuregruppen enthalten, die durch Einpolymerisieren von Carbonsäurediolen wie beispielsweise Dimethylolpropansäure als Comonomere eingeführt wurden und (c) Polyurethansequenzen enthalten, die aus Polyesterdiolen und Diisocyanaten wie beispielsweise Alkylendiisocyanaten oder Isophorondiisocyanat gebildet wurden.

Geeignete Polyurethane sind die in der US 4,743,673 beschriebenen hydrophilen Polyurethane mit Carboxygruppen im Rückgrat. Diese Polyurethane sind aufgebaut aus einer Polyolkomponente, bei der es sich um ein Alkylenglycol, ein Polyoxyalkylenglycol oder um ein lineares Polyesterdiol handeln kann, einer Carbonsäureesterkomponente mit Hydroxy- oder Aminogruppen und einem organischen Isocyanat, Polyisocyanat oder Isocyanatvorläufer, wobei die nach Polymerisation an das Polymerrückgrat gebundenen Estergruppen anschließend verseift werden.

Weitere geeignete Polyurethane sind die in der EP 0 779 310 beschriebenen, in Wasser dispergierbaren oder löslichen anionischen Polyurethane. Diese Polyurethane sind aufgebaut aus a) mindestens einer Verbindung, die zwei oder mehrere aktive Wasserstoffe pro Molekül enthält, b) mindestens einem Säure- oder Salzgruppen enthaltenden Diol und c) mindestens einem Diisocyanat. Als Komponente a) kommen vorzugsweise Polyethylenglykol, Neopentylglycol und Polyesterole, als Komponente b) vorzugsweise Dimethylolpropansäure, ein Kondensat aus Pyromellitsäuredianhydrid und Neopentylglykol sowie ein Kondensat aus 5-Natriumsulfonatoisophtalsäure mit Neopentylglykol zur Anwendung.

Weitere geeignete Polyurethane sind die in der WO 94/13724 beschriebenen kationischen Polyurethane. Diese Polyurethane sind aufgebaut aus a) mindestens einem Diisocyanat, welches bereits vorher mit einer oder mehreren Verbindungen, die zwei oder mehrere aktive Wasserstoffatome pro Molekül enthalten, umgesetzt sein kann und b) mindestens einem Diol, einem primären oder sekundären Aminoalkohol, einem primären oder sekundären Diamin oder einem primären oder sekundären Triamin mit einem oder mehreren tertiären, quartären oder protonierten tertiären Aminstickstoffatomen.

Ferner sind auch die in der EP 0745 373 genannten Polyurethane erfindungsgemäß geeignet. Diese Polyurethane besitzen neben mindestens einer Polyurethansequenz mindestens eine hydrophile Sequenz wie beispielsweise eine Polyoxyalkylensequenz und mindestens eine hydrophobe Sequenz wie beispielsweise eine Fettsäurekette mit 8 bis 30 Kohlenstoffatomen.

Erfindungsgemäß bevorzugt einzusetzende Polyurethane sind die in der US 5,626,840 beschriebenen Polyurethane mit Carboxylatgruppen. Diese Polyurethane enthalten als Verbindung, welche Carboxylatgruppen zur Verfügung stellt, eine Verbindung der Formel A-C(CH$_2$OH)$_2$-COOH worin A für ein Wasserstoffatom oder eine C1- bis C20-Alkylgruppe steht. Zumindest ein Teil der Carbonsäuregruppen wird dabei mit einer organischen oder anorganischen Base neutralisiert, um die Anzahl an Carboxylatgruppen zur Verfügung zu stellen, die erforderlich ist, um das Polyurethan in Wasser oder einem Gemisch aus Wasser und einem polaren organischen Lösungsmittel löslich zu machen.

Weitere, erfindungsgemäß bevorzugt einzusetzende Polyurethane sind die in der WO 97/17386 beschriebenen wasserlöslichen oder wasserdispergierbaren Polyurethane oder deren Salze. Diese Polyurethane werden gebildet aus a) einem in Wasser löslichen oder dispergierbaren Polyurethanpräpolymer mit endständigen Isocyanatgruppen und b) mindestens einem primären oder sekundären Amin, das wenigstens eine ionogene bzw. ionische Gruppe aufweist. Besonders bevorzugt sind die gemäß Beispiel 1 der WO 97/17386 erhältlichen Polyurethane.

Das erfindungsgemäße Mittel liegt vorzugsweise in Form einer wäßrigen oder wäßrig-alkoholischen Lösung vor. Das erfindungsgemäße Mittel enthält vorzugsweise 10 bis 80 Gewichtsprozent, besonders bevorzugt 30 bis 70 Gewichtsprozent mindestens eines Alkohols mit 1 bis 4 Kohlenstoffatomen sowie vorzugsweise 20 bis 90 Gewichtsprozent, besonders bevorzugt 30 bis 70 Gewichtsprozent Wasser.

Der Gegenstand der vorliegenden Erfindung ist hervorragend dazu geeignet, um Formulierungen mit einem reduzierten Anteil an leicht flüchtigen organischen Bestandteilen (VOC's), beispielsweise VOC 80% oder VOC 55% Formulierungen herzustellen. Das erfindungsgemäße Mittel enthält daher vorzugsweise maximal 80 Gewichtsprozent, besonders bevorzugt maximal 55 Gewichtsprozent leicht flüchtige organische Bestandteile.

Das erfindungsgemäße Haarbehandlungsmittel kann insbesondere ein Haarfestigungsmittel sein und kann unter anderem in einer Aerosolzubereitung als nichtschäumendes Spray oder als Non-Aerosol, welches mittels einer Pumpe versprüht wird, oder als „Pump and Spray" vorliegen.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Wenn das erfindungsgemäße Mittel in Form eines Aerosol-Haarsprays oder Aerosol-Haarlackes vorliegt, so enthält es zusätzlich vorzugsweise 15 bis 85 Gewichtsprozent, besonders bevorzugt 25 bis 75 Gewichtsprozent eines Treibmittels und wird in einen Druckbehälter abgefüllt. Als Treibmittel sind beispielsweise leicht flüchtige Fluorkohlenwasserstoffe oder niedere Alkane wie zum Beispiel n-Butan, i-Butan und Propan, Dimethylether sowie ferner bei den in Betracht kommenden Drücken gasförmig vorliegende Treibmittel wie beispielsweise N$_2$, N$_2$O und CO$_2$ sowie Gemische der vorstehend genannten Treibmittel geeignet.

Das erfindungsgemäße Mittel kann auch als färbendes oder pflegendes Haabehandlungsmittel wie zum Beispiel als Farbfestiger oder Pflegefestiger formuliert sein.

Das erfindungsgemäße Haarbehandlungsmittel kann weitere, übliche kosmetische Zusatzstoffe enthalten, zum Beispiel nichtfestigende, nichtionische Polymere wie Polyethylenglykol mit einem Molekulargewicht von etwa 600 g/mol, nichtfestigende, anionische Polymere sowie zusätzliche, insbesondere nichtfestigende natürliche Polymere sowie deren Mischungen in einer Menge von vorzugsweise 0,01 bis 50 Gewichtsprozent. Auch Parfümöle in einer Menge von 0,01 bis 5 Gewichtsprozent, Trübungsmittel wie Ethylenglykoldistearat in einer Menge von 0,01 bis 5 Gewichtsprozent, Netzmittel oder Emulgatoren aus den Klassen der anionischen, kationischen, amphoteren oder nichtionogenen Tenside wie Fettalkoholsulfate, ethoxylierte Fettalkohole, Fettsäurealkanolamide wie die Ester der hydrierten Rizinusölfettsäuren in einer Menge von 0,1 bis 30 Gewichtsprozent, außerdem Feuchthaltemittel, Farbstoffe, Lichtschutzmittel, Antioxidantien und Konservierungsstoffe in einer Menge von 0,01 bis 10 Gewichtsprozent können enthalten sein.

Unter Haarbehandlung soll die Behandlung des menschlichen Kopfhaares vor allem zum Zweck der Herstellung einer Frisur oder zur Pflege der Haare verstanden werden.

Desweiteren ist mit der erfindungsgemäßen Polymerkombination die Herstellung von Konzentraten möglich, welche einen verringerten Wassergehalt und/oder Lösungsmittelgehalt aufweisen. Die Konzentrate werden nach Transport und gegebenenfalls nach Lagerung durch Zugabe der erforderlichen Menge Wasser und/oder Lösungsmittel in ein anwendungsfähiges Haarbehandlungsmittel überführt.

Durch die erfindungsgemäße Kombination der Komponenten (A) und (B) werden die Nachteile der Polyurethane ausgeglichen und bei einem Einsatz in wässrigen oder wässrig-alkoholischen Haarsprays die Eigenschaften von rein alkoholischen Haarsprays erreicht und zum Teil übertroffen, was durch keines der Polymere allein möglich ist.

Die nachfolgenden Beispiele sollen den Gegenstand der Erfindung näher erläutern:

**Beispiele**

**Beispiel 1: VOC 55% Haarspray Aerosol extra starker Halt**

| | |
|---|---|
| 5,0 g | Polyurethan gemäß Beispiel 1 der WO 97/17386 |
| 2,5 g | Schellack, zu 100% neutralisiert mit Ammoniak |
| 20,0 g | Ethanol |
| 0,2 g | Parfümöl |
| 35,0 g | Dimethylether |
| 37.3 g | Wasser |
| 100,0 g | |

**Beispiel 2: VOC 80% Haarspray Aerosol starker Halt**

| | |
|---|---|
| 4,0 g | Polyurethan (Luviset® QD der Firma BASF/Deutschland) |
| 1,0 g | Oligosaccharidgemisch (C-Pur 01924, der Firma Cerestar) |
| 30,0 g | Ethanol |
| 0,2 g | Parfümöl |
| 50,0 g | Dimethylether |
| 14.8 g | Wasser |
| 100,0 g | |

**Beispiel 3: VOC 55% Haarspray Aerosol starker Halt**

| | |
|---|---|
| 3,0 g | Polyurethan gemäß Beispiel A der US 5 626 840 |
| 2,0 g | Schellack, zu 100% neutralisiert mit Ammoniak |
| 20,0 g | Ethanol |
| 0,2 g | Parfümöl |
| 35,0 g | Dimethylether |
| 39.8 g | Wasser |
| 100,0 g | |

**Beispiel 4: VOC 55% Haarspray Non Aerosol starker Halt**

| | |
|---|---|
| 5,0 g | Polyurethan gemäß Beispiel A der US 5 626 840 |
| 2,0 g | Schellack (100% neutralisiert mit Ammoniak) |
| 0,1 g | Chitosan (100% neutralisiert mit Ameisensäure) |
| 20,0 g | Ethanol |
| 0,2 g | Parfümöl |
| 72.7 g | Wasser |
| 100,0 g | |

**Beispiel 5: VOC 80% Haarspray Non-Aerosol starker Halt für feines Haar**

| | |
|---|---|
| 4,0 g | Polyurethan gemäß Beispiel 1 der WO 97/17386 |
| 2,0 g | Schellack, zu 80% neutralisiert mit Ammoniak |

| 80,0 g | Ethanol |
| 0,2 g | Parfümöl |
| 13.8 g | Wasser |
| 100,0 g | |

**Bruchkraftmessungen**

1. Untersuchte Zusammensetzungen

Es wurden vier Zusammensetzungen A bis D untersucht. Zusammensetzungen A bis C enthalten eine Kombination von Polyurethan und Schellack in verschiedenen Mengenverhältnissen. Zusammensetzung D enthält als einziges Polymer Polyurethan. Die genauen Zusammensetzungen sind in Tabelle 1 aufgeführt.

Tabelle 1

| Untersuchte Zusammensetzungen | | | | |
|---|---|---|---|---|
| Inhaltsstoff | A | B | C | D |
| Polyurethan Luviset® QD (BASF) | 6 g | 7 g | 8 g | 9 g |
| Schellack, zu 100% neutralisiert mit Natriumtetraborat | 3 g | 2 g | 1 g | - |
| Ethanol | 54,4 g | 54,3 g | 54,2 g | 54,1 g |
| Wasser | 36,6 g | 36,7 g | 36,8 g | 36,9 g |

2. Untersuchungsmethode

Standardisierte Haarsträhnen mit einheitlichem Gewicht und einheitlicher Länge von 7,5 cm (Zählhaarsträhnen der Fa. Kerling) wurden mit einem Shampoo gewaschen, mit Wasser gespült und getrocknet. Danach wurden die Strähnen in eine Folientasche gelegt, mit 65 $\mu$l Wasser pro Strähne beträufelt und anschließend mit je 50 $\mu$l der Haarspraylösung beträufelt. Die Folientaschen werden geschlossen und nach einer Einwirkzeit von 15 Minuten werden alle Strähnen auf einer glatten Unterlage im Klimaraum getrocknet. Danach wurden für jede Strähne je drei Bruchkraftmessungen durchgeführt. Insgesamt wurden für jede Lösung drei Strähnen untersucht. Die Bruchkraft ist ein Maß für die Haarfestigung.

Die Messungen wurden mit einer Bruchkraft-Meßapparatur durchgeführt. Hierbei befindet sich ein Haken unterhalb der Mitte einer Haarprobe. Die Haarprobe wird an den Enden festgehalten und durch den Haken in der Mitte langsam mit einer konstanten Geschwindigkeit mittels eines Schrittmotors nach oben gezogen. Gleichzeitig wird die Kraft, die benötigt wird, um die Haarsträhne nach oben zu ziehen, mit einem Wägezellen-Kraftaufnehmer (Typ Q11 der Firma Hottinger Baldwin Messtechnik) gemessen. Das Signal des Kraftaufnehmers wurde verstärkt und per Computer aufgezeichnet. Die Kraft, die gebraucht wird, um die Strähne in der Mitte hochzuziehen, steigt hierbei solange kontinuierlich an, bis der auf dem Haar befindliche Polymerfilm bricht und fällt anschließend wieder. Das beobachtete Maximum der Kraftmessung entspricht der Bruchkraft.

3. Ergebnisse

Die Ergebnisse der Bruchtkraftmessungen sind in Tabelle 2 dargestellt und stellen jeweils Mittelwerte von 9 Messungen dar.

Tabelle 2

| Ergebnisse der Bruchkraft-messungen | |
|---|---|
| Muster | Bruchkraft |
| A | 0,425 N |
| B | 0,394 N |

Tabelle 2 (fortgesetzt)

| Ergebnisse der Bruchkraft-messungen | |
|---|---|
| Muster | Bruchkraft |
| C | 0,366 N |
| D | 0,299 N |

Die Muster zeigen eine deutliche Erhöhung der Bruchkraft für die einen Zusatz von Schellack enthaltenden Muster A, B und C.

**Patentansprüche**

1. Verwendung von Polymeren natürlichen Ursprungs zur Verbesserung von Filmbildungseigenschaften von Polyurethanen.

2. Verwendung von Polymeren natürlichen Ursprungs zur Herstellung eines Polyurethan enthaltenden Faserbehandlungsmittels.

3. Verwendung nach Anspruch 2, dadurch gekennzeichnet, daß es sich bei den Fasern um Keratinfasern, insbesondere um Haare handelt.

4. Verwendung nach einem der vorstehenden Ansprüche, dadurch gekennzeichnet, daß das Polymer natürlichen Ursprungs ausgewählt ist aus Schellack, Chitosan, alkoxylierten Chitosanen, alkoxylierten Chitinen, Polysacchariden und chinesischem Balsamharz.

5. Verwendung nach einem der vorstehenden Ansprüche, dadurch gekennzeichnet, daß das Polyurethan ausgewählt ist aus hydrophilen, wasserlöslichen oder wasserdispergierbaren Polyurethanen mit ionogenen oder ionischen Gruppen.

6. Haarbehandlungsmittel mit einem Gehalt an

   (A) mindestens einem Polyurethan und
   (B) mindestens einem Polymer natürlichen Ursprungs

7. Mittel nach Anspruch 6, dadurch gekennzeichnet, daß das Polyurethan ausgewählt ist aus hydrophilen, wasserlöslichen oder wasserdispergierbaren Polyurethanen mit ionogenen oder ionischen Gruppen.

8. Mittel nach Anspruch 6, dadurch gekennzeichnet, daß das Polyurethan ausgewählt ist aus mindestens einer der Gruppen i) bis v)

   i) Polyurethane, gebildet aus einer Polyolkomponente, bei der es sich um ein Alkylenglycol, ein Polyoxyalkylenglycol oder um ein lineares Polyesterdiol handeln kann, einer Carbonsäureesterkomponente mit Hydroxy- oder Aminogruppen und einem organischen Isocyanat oder Isocyanatvorläufer, wobei die nach Polymerisation an das Polymerrückgrat gebundenen Estergruppen verseift sind;
   ii) Polyurethane, aufgebaut aus a) mindestens einer Verbindung, die zwei oder mehrere aktive Wasserstoffe pro Molekül enthält, b) mindestens einem Säure- oder Salzgruppen enthaltenden Diol und c) mindestens einem Diisocyanat;
   iii) Polyurethane, aufgebaut aus a) mindestens einem Diisocyanat, welches bereits vorher mit einer oder mehreren Verbindungen, die zwei oder mehrere aktive Wasserstoffatome pro Molekül enthalten, umgesetzt sein kann und b) mindestens einem Diol, einem primären oder sekundären Aminoalkohol, einem primären oder sekundären Diamin oder einem primären oder sekundären Triamin mit einem oder mehreren tertiären, quartären oder protonierten tertiären Aminstickstoffatomen;
   iv) Polyurethane mit Carboxylatgruppen und einem Gehalt an Monomer-Diolen der Formel $A$-$C(CH_2OH)_2$-$COOH$ worin A für ein Wasserstoffatom oder eine C1- bis C20-Alkylgruppe steht;
   v) Polyurethane, gebildet aus a) einem in Wasser löslichen oder dispergierbaren Polyurethanpräpolymer mit endständigen Isocyanatgruppen und b) mindestens einem primären oder sekundären Amin, das wenigstens eine ionogene bzw. ionische Gruppe aufweist.

9. Mittel nach Anspruch 8, dadurch gekennzeichnet, daß das Polyurethan ausgewählt ist aus der Gruppe iv) oder v).

10. Mittel nach einem der Ansprüche 6 bis 9, dadurch gekennzeichnet, daß das Polymer natürlichen Ursprungs ausgewählt ist aus Schellack, Chitosan, alkoxylierten Chitosanen und Chitinen, Polysacchariden und chinesischem Balsamharz.

11. Mittel nach einem der Ansprüche 6 bis 10, dadurch gekennzeichnet, daß das Polymer natürlichen Ursprungs Schellack ist.

12. Mittel nach einem der Ansprüche 6 bis 11, dadurch gekennzeichnet, daß Komponente (A) in 0,1 bis 20 Gewichtsprozent enthalten ist.

13. Mittel nach einem der Ansprüche 6 bis 12, dadurch gekennzeichnet, daß Komponente (B) in 0,1 bis 20 Gewichtsprozent enthalten ist.

14. Mittel nach einem der Ansprüche 6 bis 13 in Form einer niedrig-viskosen, wäßrigen oder wäßrig-alkoholischen Lösung.

15. Mittel nach einem der Ansprüche 6 bis 14, dadurch gekennzeichnet, daß es entweder ein Treibmittel enthält und in Form eines Aerosol Haarsprays vorliegt oder in Kombination mit einer mechanischen Pumpvorrichtung als Pumpspray vorliegt.

16. Mittel nach einem der Ansprüche 6 bis 15, dadurch gekennzeichnet, daß es weniger als 80 Gewichtsprozent flüchtige organische Bestandteile enthält.